# EUROPEAN PATENT SPECIFICATION

(11) **EP 1 029 079 B1**
(45) Date of publication and mention of the grant of the patent: **09.01.2008**
(21) Application number: 98957658.2
(22) Date of filing: 09.11.1998
(51) Int. Cl.: C12Q 1/68

(54) **DIAGNOSTICS AND THERAPEUTICS FOR CHRONIC OBSTRUCTIVE AIRWAY DISEASE**
RISIKOABSCHÄTZUNG FÜR CHRONISCHE ATEMWEGSERKRANKUNGEN UND ANWENDUNGEN
DIAGNOSTICS ET THERAPIES DES MALADIES OBSTRUCTIVES CHRONIQUES DES VOIES RESPIRATOIRES

(30) Priority: 07.11.1997 GB 9723553; 12.01.1998 US 5923
(43) Date of publication of application: 23.08.2000
(73) Proprietor: Interleukin Genetics, Inc., Cincinnati, OH 45208 (US)
(72) Inventor: DUFF, Gordon, W., Sheffield, South Yorkshire S10 3B2 (GB); GIOVINE, Marco, Sheffield, South Yorkshire S10 3B2 (GB); BARNES, Peter, Imp.Coll.School of Medicine ., London SW3 6LY (GB); LIM, Simon, Imperial Coll.School of Medicine ., London SW3 6LY (GB)
(74) Representative: Crump, Julian Richard John
(86) International application number: PCT/US1998/023721
(87) International publication number: WO 1999/024615

(56) References cited:
- WO-A-95/01997
- WO-A-97/06180
- WO-A-98/54359
- US-A- 5 674 483
- MCDOWELL T L ET AL: "A gentic association between juvenile rheumatoid arthritis and a novel interleukin-1 alpha polymorphism" ARTHRITIS AND RHEUMATISM, vol. 2, no. 38, 1900, page 221 228 XP002077314 ISSN: 0004-3591

## Description

### 1. BACKGROUND OF THE INVENTION

Asthma is a chronic lung disease characterized by coughing, chest tightness, shortness of breath, and wheezing due to a reversible obstruction of airflow resulting from inflammation and hyper-responsiveness of the airways. In sensitized individuals, inhalation of allergens may produce inflammation of the airway lining, and precipitate a flare-up of asthma. Asthma may also occur as a result of other inflammatory stimuli, such as respiratory tract infections. Individuals who have become sensitized to specific foods may have severely and possibly life-threatening reactions after ingestion of these substances. Asthma, once thought of as a "simple" hypersensitivity reaction, is now known to be a complex condition with a probable spectrum of causes and contributing factors, with airway inflammation as its central attribute. Pulmonary researchers liken it to arteriosclerosis, in the sense that there are many interactive aspects. Many of the contributing factors are now under intensive study, including the chemical reactions that take place in the asthmatic process; the nature of cell-cell communication, the way information is conveyed from one cell or type of cell to another; and the role, reactive or other, of the epithelium. Allergies contribute to both the incidence and severity of asthmatic symptoms. An allergy (also known as immediate hypersensitivity) is defined as an abnormal sensitivity to a substance which is normally tolerated and generally considered harmless, and for which the triggering event is dose-independent, as opposed to a dose-dependent idiosyncratic reaction to a substance. While all immune responses occur as a result of exposure to foreign substances, allergic reactions are distinct from the protective or enhanced "immunity" conferred by immunizations or natural infection. Only about a quarter of the children with asthma outgrow the condition when their airways reach adult size; for the rest, the condition is a lifelong ordeal. The condition persists, according to a research report published by the American Lung Association, in 85 percent of women and in 72 percent of men. (Journal of Allergy and Clinical Immunology Vol. 96:5 11/96).

There were 4,964 deaths from asthma recorded in 1993 in the United States alone. The incidence of asthma mortality in children doubled from 1980 to 1993. Among persons between the ages of 15 and 24 years, the number of deaths rose from 2.5 cases per million in 1980 to 5.2 cases per million in 1993. In 1993, asthma accounted for 342 deaths and approximately 198,000 hospitalization in persons under 25 years of age.

African-Americans account for 21 percent of deaths due to asthma. African-American children are four times more likely to die of asthma than Caucasian children. African-American males between the ages of 15 and 24 have the highest risk of mortality.

A positive family history tends to be one of the strongest risk factors associated with asthma. Positive identification though, can be difficult. Asthma may coexist with other conditions such as congenital abnormalities, infectious conditions, and cystic fibrosis. Additional indicators are considered when the history is atypical or the response to good medical management is poor. Physicians with less experience in the management of this disease may treat these symptoms as an infection, not realizing that the underlying cause is asthma.

The identification of asthma in children relies heavily on the parents' observations for clinical clues. Correct identification requires an asthma and allergy specialist who recognizes the uniqueness of childhood asthma. More subtle signs of asthma, such as chest tightness, may be overlooked, particularly by children. Recurrent or constant coughing spells may be the only common observable symptoms of asthma in young children. Although, demonstration of a favorable clinical response to bronchodilator therapy can help confirm the presence of asthma.

There is a tremendous need for early identification of those who are generally susceptible to asthma. Because COAD is a chronic and progressive disease when untreated, early identification would facilitate the administration of appropriate treatment at the earliest stage, thereby increasing the probability of a positive outcome

### 2. SUMMARY OF THE INVENTION

In one aspect, the invention features assays for determining a subject's susceptibility to developing asthma.

In one embodiment, the method comprises the step of genotyping a nucleic acid sample obtained from blood, saliva, skin or hair of the subject to determine at least one allele of an IL-1 proinflammatory haplotype, detecting at least IL-1B allele 2 (-511) containing a T at IL-1B base -511 or IL-1B allele 2 (+3954) containing a T at IL-1B base +3954 in the sample, wherein detection of said allele indicates that the patient has an increased risk of susceptibility to developing asthma.

For example, an allele of an IL-1 proinflammatory haplotype can be detected by: 1) performing a hybridization reaction between the nucleic acid sample and a probe or probes that are capable of hybridizing to an allele of an IL-1 haplotype in the subject; 2) sequencing at least a portion of at least one allele of an IL-1 haplotype, or 3) determining the electrophoretic mobility of at least one allele of an IL-1 haplotype or a component thereof In another preferred embodiment, a component of an IL-1 haplotype is subject to an amplification step, prior to performance of the detection step. Preferred amplification steps are selected from the group consisiting of the polymerase chain reaction (PCR), the ligase chain reaction (LCR), strand displacement amplification (SDA), cloning, and variations of the above (e.g. RT-PCR and allele specific amplification). In a particularly preferred embodiment, the sample is hybridized with a set of primers, which hybridize 5' and 3' to a sense or antisense sequence of the allele of the IL-1 haplotype and is subject to a PCR amplification.

Also disclosed are kits for performing the above-described assays. The kit can include DNA sample collection means and a means for determining at least one allele of an IL-1 haplotype of the subject. The kit may also comprise control samples or standards.

Information obtained using the assays and kits described herein (alone or in conjunction with information on another genetic defect or environmental factor, which contributes to chronic obstructive airway disease) is useful for predicting whether a subject is likely to develop chronic obstructive airway disease. In addition, the information alone or in conjunction with information on another genetic defect contributing to chronic obstructive airway disease (the genetic profile of chronic obstructive airway disease) allows customization of therapy to the individual's genetic profile. For example, this information can enable a doctor to: 1) more effectively prescribe a drug that will address the molecular basis of the cascade resulting in chronic obstructive airway disease; and 2) better determine the appropriate dosage of a particular drug for the particular patient. The ability to target patient populations expected to show
the highest clinical benefit, can enable: 1) the repositioning of marketed drugs with disappointing market results; 2) the rescue of drug candidates whose clinical development has been discontinued as a result of safety or efficacy limitations, which are patient subgroup-specific; and 3) an accelerated and less costly development for drug candidates and more optimal drug labeling.

Other features and advantages of the invention will be apparent from the following detailed description and claims.

### 3. BRIEF DESCRIPTION OF THE DRAWINGS

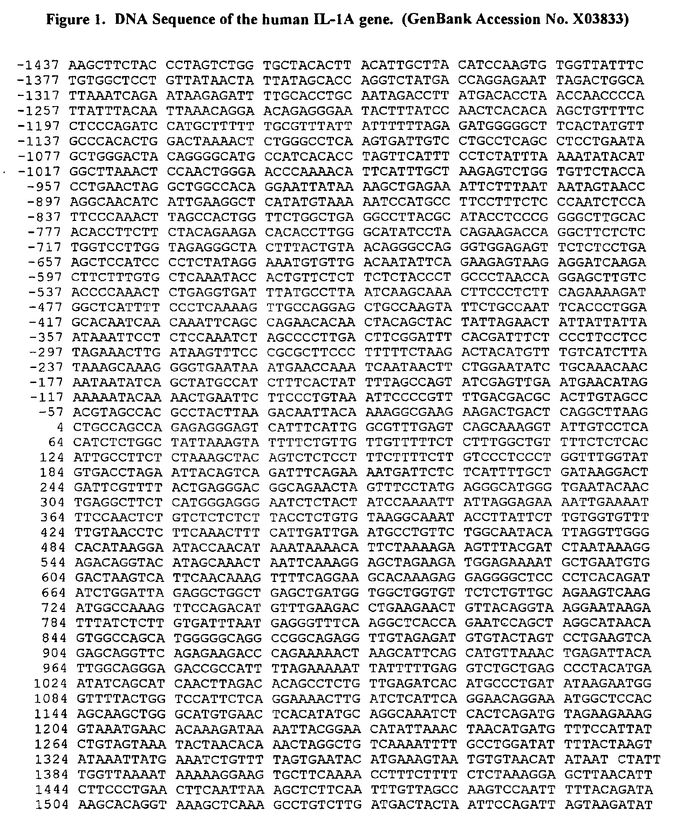

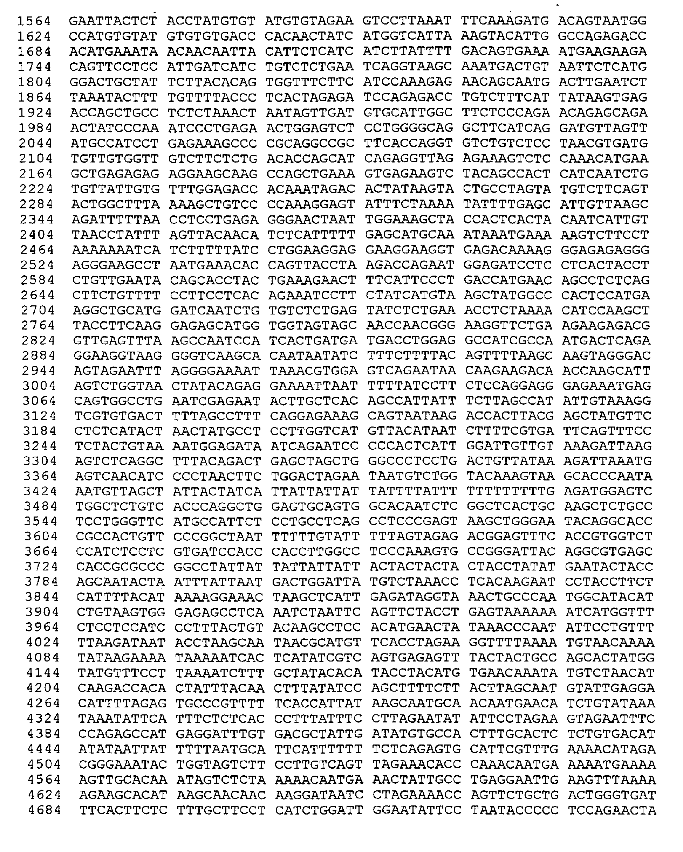

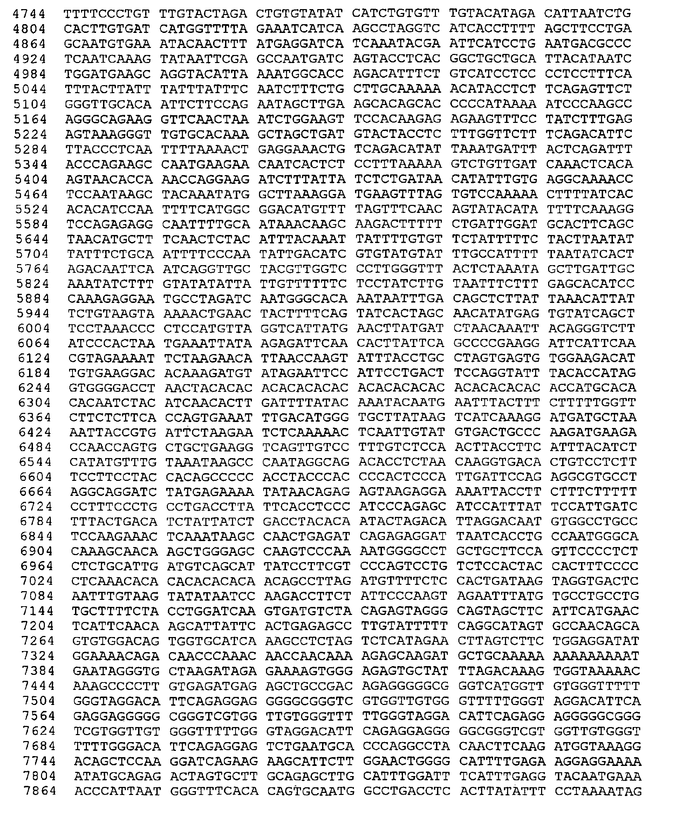

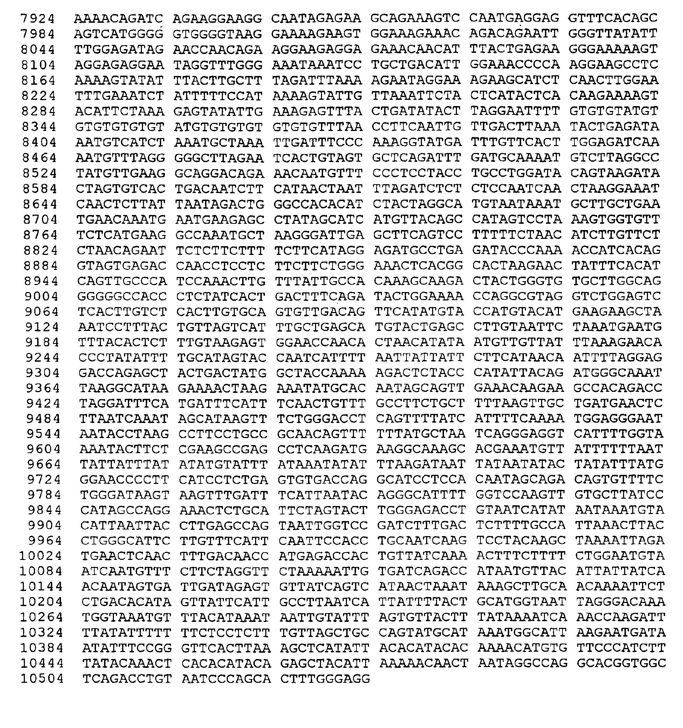

Figure 1 shows the DNA sequence of the human IL-1A gene (GenBank Accession No. X03833).

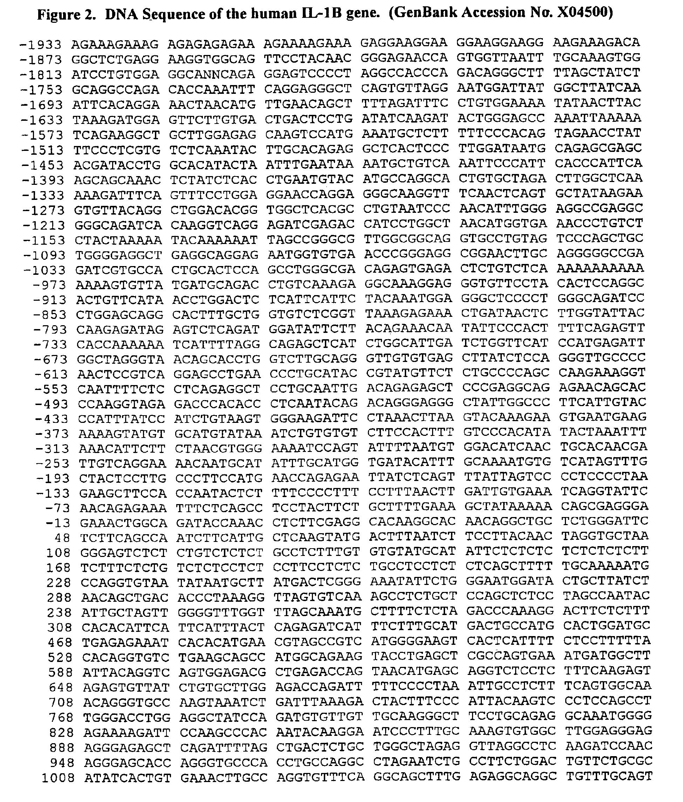

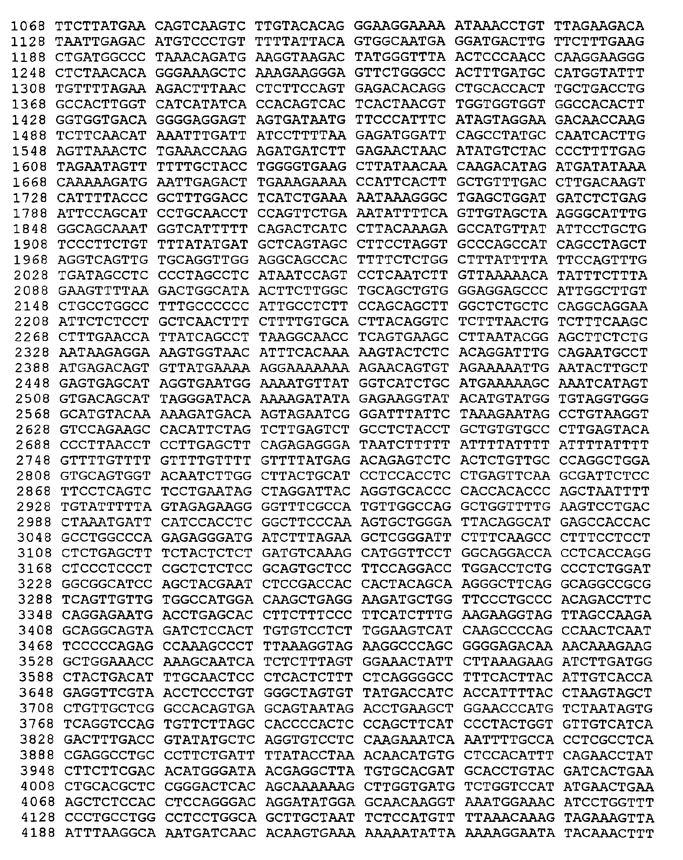

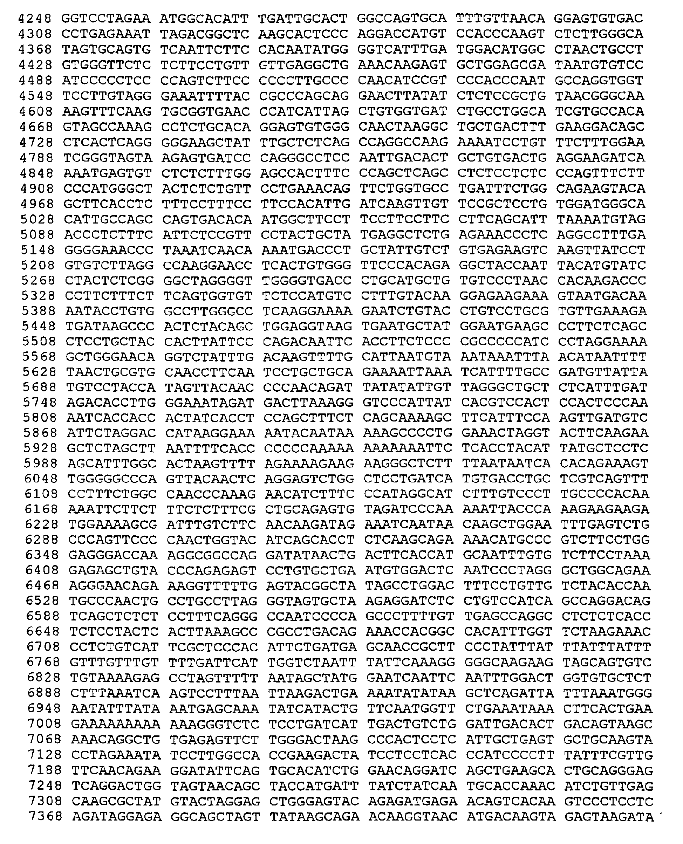

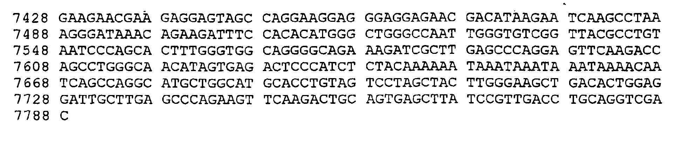

Figure 2 shows the DNA sequence of the human IL-1B gene (GenBank Accession No. X04500).

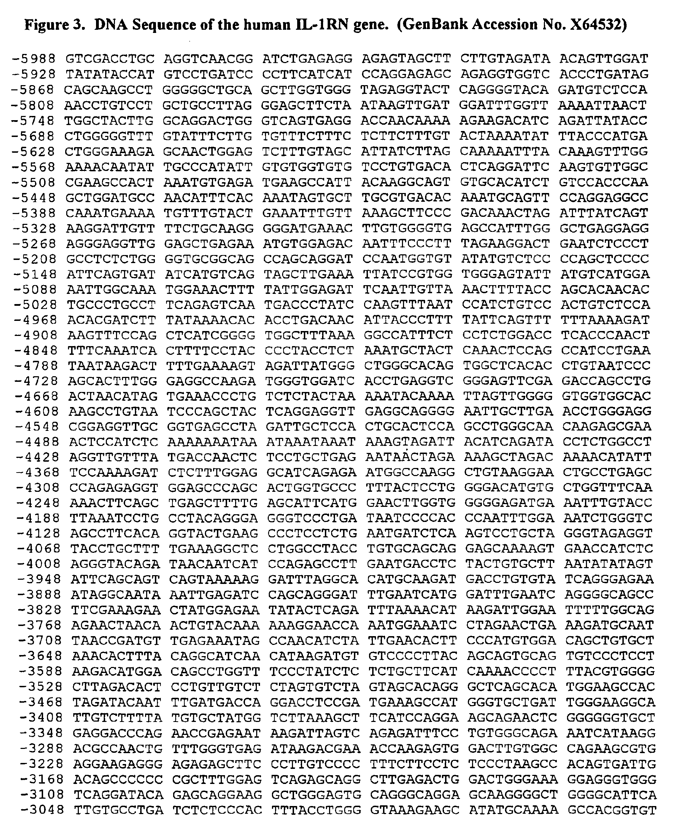

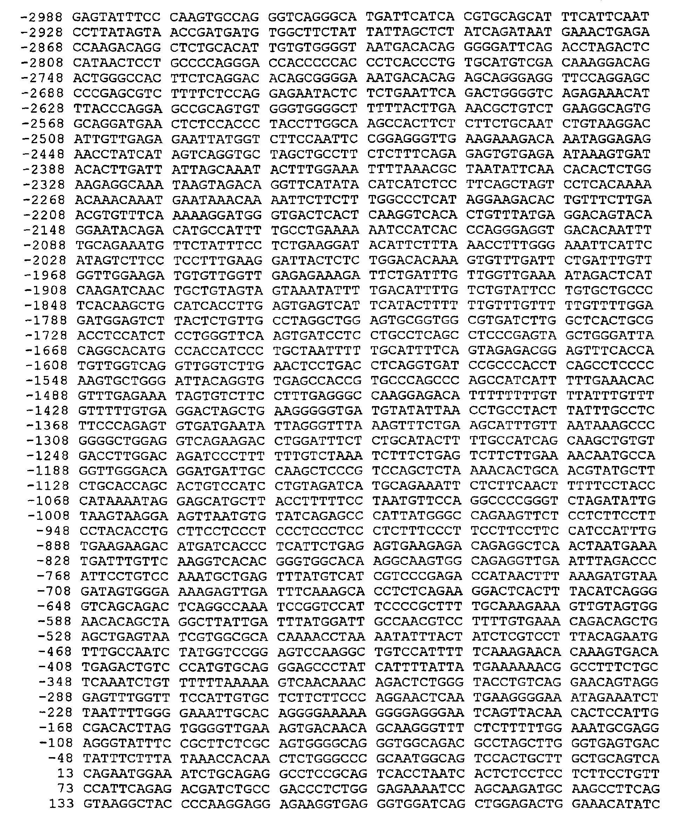

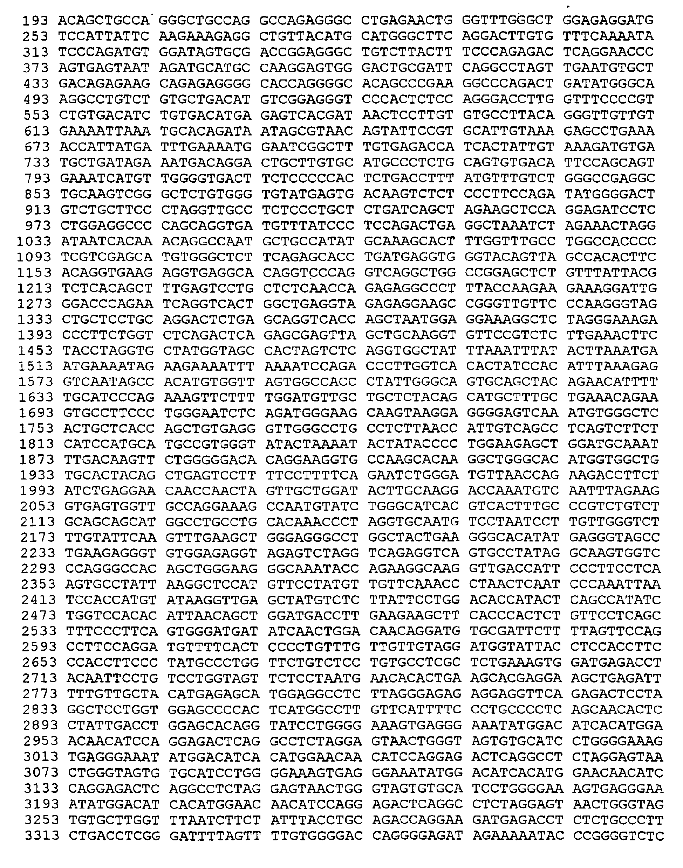

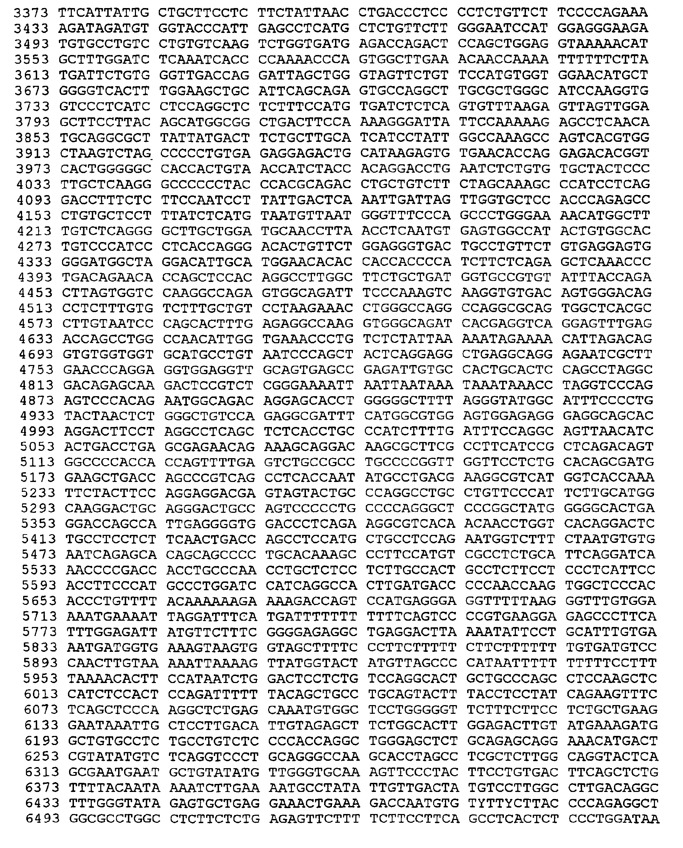

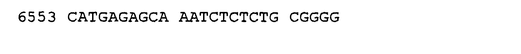

Figure 3 shows the DNA sequence of the human IL1-RN gene (GenBank Accession No. X64532).

### 4. DETAILED DESCRIPTION OF THE INVENTION

### 4.1 Definitions

For convenience, the meaning of certain terms and phrases employed in the specification, examples, and appended claims are provided below.

The term " allele" refers to alternative forms of a gene at a particular marker. When a subject has two identical alleles, the subject is said to be homozygous. When a subject has two different alleles, the subject is said to be heterozygous.

"Chronic obstructive lung disease" or "chronic obstructive airway disease" (COAD) are terms used to describe a complex of conditions that have in common airflow limitation or airflow obstruction. COAD includes asthma, emphysema, chronic bronchitis, and chronic bronchiolitis. The sites of airway obstruction in COADs vary from the upper airways to the most peripheral bronchioles. The exact cause of most diseases of the airways is not well understood. The definition of airway diseases add to the confusion. Chronic bronchitis is defined clinically by the chronic presence of cough and sputum production. Emphysema, on the other hand, is defined anatomically, on the basis of the breakdown of lung tissue and the enlargement of the alveolar sacs. COADs all have airway narrowing as a disease parameter and they also share inflammation as a component of the disease process.

"Genotyping" refers to the analysis of an individual's genomic DNA (or a nucleic acid corresponding thereto) to identify a particular disease causing or contributing mutation or polymorphism, directly or based on detection of a mutation or polymorphism (a marker) that is in linkage disequilibrium with the disease causing or contributing gene.

The term "haplotype" refers to a set of alleles that are inherited together as a group (are in linkage disequilibrium). As used herein, haplotype is defined to include those haplotypes that occur at statistically significant levels (p_{corr} ≤ 0.05). As used herein, the phrase "an IL-1 haplotype" refers to a haplotype in the IL-1 loci including alleles of the IL-1A, IL-1B and IL-1RN genes and markers in disequilibrium therewith. "Proinflammatory IL-1 haplotype" refers to a haplotype that is associated with an excess of proinflammatory release and/or activity (e.g upregulation of functional IL- 1α and/or IL-1β and/or downregulation of a functional IL-1 receptor antagonist).

"Linkage disequilibrium" refers to co-inheritance of two alleles at frequencies greater than would be expected from the separate frequencies of occurrence of each allele in a given control population. The expected frequency of occurrence of two alleles that are inherited independently is the frequency of the first allele multiplied by the frequency of the second allele. Alleles that co-occur at expected frequencies are said to be in "linkage equilibrium".

Examples of polymorphic markers in linkage disequilibrium include: the IL-1B allele 2 (-511), IL-1A allele 4(222/223), IL-1A allele 1(gz5/gz6), IL,-1A allele 1(-889), the IL-1B allele 2 (+6912), IL-1B allele 1 (+3954), IL-1B/ IL-1RN intergenic region (gaat.p33330) and (Y31), IL-1RN allele 2 (+2018), IL-1RN allele 2 (VNTR) and three polymorphisms that are in linkage disequilibrium with IL-1RN allele 2 (VNTR), (See Clay et al., (1996) Hum Genet 97:723-726).

The term "polymorphism" refers to the coexistence of more than one form of a gene or portion (e.g., allelic variant) thereof A portion of a gene of which there are at least two different forms, i.e., two different nucleotide sequences, is referred to as a "polymorphic region of a gene". A polymorphic region can be a single nucleotide, the identity of which differs in different alleles. A polymorphic region can also be several nucleotides long.

### 4.2 Predictive Medicine

### 4.2.1. Prognostic Assays and Kits

Based on the findings described in detail in the following examples, the IL-1B allele 2 (+3954) and IL-1B allele 2 (-511) are significantly associated with asthma, the present invention provides methods for determining whether a subject has or is likely to develop asthma and/or for predicting the extent or progression of asthma in a subject.

In one embodiment, the method comprises genotyping a nucleic acid sample obtained from blood, saliva, skin or hair of the subject to detect at least one allele of IL-1 proinflammatory haplotypes IL-1B allele 2(-511) or IL-1B allele 2(+3954). For example, an allele of an IL-1 proinflammatory haplotype can be detected, for example, by determining the transcription rate or mRNA and/or protein level of an IL-1 gene or protein, such as by Northern blot analysis, reverse transcription-polymerase chain reaction (RT-PCR), *in situ* hybridization, immunoprecipitation, Western blot hybridization, or immunohistochemistry. According to one method, cells are obtained from a subject and the IL-1 protein or mRNA level is determined and compared to the level of IL-1 protein or mRNA level in a healthy subject.

In preferred embodiments, the method is characterized as comprising genotyping a nucleic acid sample obtained from blood, saliva, skin or half of the subject to determine at least one allele of IL-1 proinflammatory haplotype IL-18 allele 2 (-511) or IL-18 allele 2 (+3954). In an exemplary embodiment, there is provided a nucleic acid composition comprising a nucleic acid probe including a region of nucleotide sequence which is capable of hybridizing to a sense or antisense sequence of the at least one allele of an IL-1 proinflammatory haplotype IL-18 allele 2 (-511) or IL-1B allele 2 (+3954). For example, the nucleic acid can be rendered accessible for hybridization, the probe contacted with the nucleic acid of the sample, and the hybridization of the probe to the sample nucleic acid detected. Such technique can be used to detect alterations or allelic variants at either the genomic or mRNA level as well as to determine mRNA transcript levels.

A preferred detection method is allele specific hybridization using probes overlapping a region of at least one allele of IL-1 proinflammatory haplotype IL-18 allele 2 (-511) or IL-18 allele 2 (+3954) and having about 5, 10, 20, 25, or 30 nucleotides around the mutation or polymorphic region. Several probes capable of hybridizing specifically to other allelic variants involved in a chronic obstructive airway disease may be attached to a solid phase support, e.g., a "chip" (which can hold up to about 250,000 oligonucleotides). Oligonucleotides can be bound to a solid support by a variety of processes, including lithography. Mutation detection analysis using these chips comprising oligonucleotides, also termed "DNA probe arrays" is described e.g., in Cronin et al. (1996) Human Mutation 7:244. A chip may comprise all the allelic variants of at least one polymorphic region of a gene. The solid phase support is then contacted with a test nucleic acid and hybridization to the specific probes is detected. Accordingly, the identity of numerous allelic variants of one or more genes can be identified in a simple hybridization experiment.

These techniques may also comprise the step of amplifying the nucleic acid before analysis. Amplification techniques are known to those of skill in the art and include, but are not limited to cloning, polymerase chain reaction (PCR), polymerase chain reaction of specific alleles (ASA), ligase chain reaction (LCR), nested polymerase chain reaction, self sustained sequence replication (Guatelli, J.C. et al., 1990, Proc. Natl. Acad. Sci. USA 87:1874-1878), transcriptional amplification system (Kwoh, D.Y. et al., 1989, Proc. Natl. Acad. Sci. USA 86:1173-1177), and Q-Beta Replicase (Lizardi, P.M. et al., 1988, Bio/Technology 6:1197).

Particularly preferred primers for use in a PCR reaction include:

5'-CTC.AGC.AAC.ACT.CCT.AT-3' (SEQ ID NO. 5)
5'-TCC.TGG.TCT.GCA.GCT.AA-3' (SEQ ID NO. 6)
5'-CTA TCT GAG GAA CAA ACT AGT AGC-3' (SEQ ID NO. 7)
5'-TAG GAC ATT GCA CCT AGG GTT TGT -3' (SEQ ID NO. 8)
5'-AAG CTT GTT CTA CCA CCT GAA CTA GGC.-3' (SEQ ID NO. 9) and
5'-TTA CAT ATG AGC CTT CCA TG.-3' (SEQ ID NO. 10)

Amplification products may be assayed in a variety of ways, including size analysis, restriction digestion followed by size analysis, detecting specific tagged oligonucleotide primers in the reaction products, allele-specific oligonucleotide (ASO) hybridization, allele specific 5' exonuclease detection, sequencing, hybridization, and the like.

PCR based detection means can include multiplex amplification of a plurality of markers simultaneously. For example, it is well known in the art to select PCR primers to generate PCR products that do not overlap in size and can be analyzed simultaneously. Alternatively, it is possible to amplify different markers with primers that are differentially labeled and thus can each be differentially detected. Of course, hybridization based detection means allow the differential detection of multiple PCR products in a sample. Other techniques are known in the art to allow multiplex analyses of a plurality of markers.

In a merely illustrative embodiment, the method includes the steps of (i) collecting a sample of cells from a patient, (ii) isolating nucleic acid (e.g., genomic, mRNA or both) from the cells of the sample, (iii) contacting the nucleic acid sample with one or more primers which specifically hybridize 5' and 3' to at least one allele of IL-1 proinflammatory haplotype IL-18 allele 2 (-511) or IL-18 allele 2 (+3954) under conditions such that hybridization and amplification of the allele occurs, and (iv) detecting the amplification product. These detection schemes are especially useful for the detection of nucleic acid molecules if such molecules are present in very low numbers.

In a preferred embodiment of the subject assay, the allele of the IL-1 proinflammatory haplotype IL-18 allele 2 (-511) or IL-18 allele 2 (+3954) is identified by alterations in restriction enzyme cleavage patterns. For example, sample and control DNA is isolated, amplified (optionally), digested with one or more restriction endonucleases, and fragment length sizes are determined by gel electrophoresis.

In yet another embodiment, any of a variety of sequencing reactions known in the art can be used to directly sequence the allele. Exemplary sequencing reactions include those based on techniques developed by Maxim and Gilbert *(Proc. Natl Acad Sci USA* (1977) 74:560) or Sanger (Sanger et al (1977) *Proc. Nat. Acad Sci* 74:5463). It is also contemplated that any of a variety of automated sequencing procedures may be utilized when performing the subject assays (*Biotechniques* (1995) 19:448), including sequencing by mass spectrometry (see, for example PCT publication WO 94/16101; Cohen et al. (1996) Adv Chromatogr 36:127-162; and Griffin et al. (1993) Appl Biochem Biotechnol 38:147-159). It will be evident to one of skill in the art that, for certain embodiments, the occurrence of only one, or two of the nucleic acid bases need be determined in the sequencing reaction. For instance, A-track or the like, e.g., where only one nucleic acid is detected, can be carried out.

In a further embodiment, protection from cleavage agents (such as a nuclease, hydroxylamine or osmium tetroxide and with piperidine) can be used to detect mismatched bases in RNA/RNA or RNA/DNA or DNA/DNA heteroduplexes (Myers, et al. (1985) Science 230:1242). In general, the art technique of "mismatch cleavage" starts by providing heteroduplexes formed by hybridizing (labelled) RNA or DNA containing the wild-type allele with the sample. The double-stranded duplexes are treated with an agent which cleaves single-stranded regions of the duplex such as which will exist due to base pair mismatches between the control and sample strands. For instance, RNA/DNA duplexes can be treated with RNase and DNA/DNA hybrids treated with S 1 nuclease to enzymatically digest the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al (1988) Proc. Natl Acad Sci USA 85:4397; Saleeba et al (1992) Methods Enzymol. 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes). For example, the mutY enzyme of *E. coli* cleaves A at G/A mismatches and the thymidine DNA glycosylase from HeLa cells cleaves T at G/T mismatches (Hsu et al. (1994) Carcinogenesis 15:1657-1662). According to an exemplary embodiment, a probe based on an allele of a proinflammatory haplotype is hybridized to a cDNA or other DNA product from a test cell(s). The duplex is treated with a DNA mismatch repair enzyme, and the cleavage products, if any, can be detected from electrophoresis protocols or the like. See, for example, U.S. Patent No. 5,459,039.

In other embodiments, alterations in electrophoretic mobility will be used to identify IL-1β allele 2 (-511). For example, single strand conformation polymorphism (SSCP) may be used to detect differences in electrophoretic mobility between mutant and wild type nucleic acids (Orita et al. (1989) Proc Natl. Acad. Sci USA 86:2766, see also Cotton (1993) Mutat Res 285:125-144; and Hayashi (1992) Genet Anal Tech Appl 9:73-79). Single-stranded DNA fragments of sample and control IL- 1β alleles (-511) are denatured and allowed to renature. The secondary structure of single-stranded nucleic acids varies according to sequence, the resulting alteration in electrophoretic mobility enables the detection of even a single base change. The DNA fragments may be labelled or detected with labelled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet 7:5).

In yet another embodiment, the movement of alleles in polyacrylamide gels containing a gradient of denaturant is assayed using denaturing gradient gel electrophoresis (DGGE) (Myers et al (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing agent gradient to identify differences in the mobility of control and sample DNA (Rosenbaum and Reissner (1987) Biophys Chem 265:12753).

Examples of other techniques for detecting alleles include, but are not limited to, selective oligonucleotide hybridization, selective amplification, or selective primer extension. For example, oligonucleotide primers may be prepared in which the known mutation or nucleotide difference (e.g., in allelic variants) is placed centrally and then hybridized to target DNA under conditions which permit hybridization only if a perfect match is found (Saiki et al. (1986) Nature 324:163); Saiki et al (1989) Proc. Natl Acad. Sci USA 86:6230). Such allele specific oligonucleotide hybridization techniques may be used to test one mutation or polymorphic region per reaction when oligonucleotides are hybridized to PCR amplified target DNA or a number of different mutations or polymorphic regions when the oligonucleotides are attached to the hybridizing membrane and hybridized with labelled target DNA.

Alternatively, allele specific amplification technology which depends on selective PCR amplification may be used in conjunction with the instant invention. Oligonucleotides used as primers for specific amplification may carry the mutation or polymorphic region of interest in the center of the molecule (so that amplification depends on differential hybridization) (Gibbs et al (1989) Nucleic Acids Res. 17:2437-2448) or at the extreme 3' end of one primer where, under appropriate conditions, mismatch can prevent, or reduce polymerase extension (Prossner (1993) Tibtech 11:238. In addition it may be desirable to introduce a novel restriction site in the region of the mutation to create cleavage-based detection (Gasparini et al (1992) Mol. Cell Probes 6:1). It is anticipated that in certain embodiments amplification may also be performed using Taq ligase for amplification (Barany (1991) Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

In another embodiment, identification of the allelic variant is carried out using an oligonucleotide ligation assay (OLA), as described, e.g., in U.S. Pat. No. 4,998,617 and in Landegren, U. et al., Science 241:1077-1080 (1988). The OLA protocol uses two oligonucleotides which are designed to be capable of hybridizing to abutting sequences of a single strand of a target. One of the oligonucleotides is linked to a separation marker, e.g,. biotinylated, and the other is detectably labeled. If the precise complementary sequence is found in a target molecule, the oligonucleotides will hybridize such that their termini abut, and create a ligation substrate. Ligation then permits the labeled oligonucleotide to be recovered using avidin, or another biotin ligand. Nickerson, D. A. et al. have described a nucleic acid detection assay that combines attributes of PCR and OLA (Nickerson, D. A. et al., Proc. Natl. Acad. Sci. (U.S.A.) 87:8923-8927 (1990), In this method, PCR is used to achieve the exponential amplification of target DNA, which is then detected using OLA.

Several techniques based on this OLA method have been developed and can be used to detect alleles of an IL-1 proinflammatory haplotype. For example, U.S. Patent No. 5,593,826 discloses an OLA using an oligonucleotide having 3'-amino group and a 5'-phosphorylated oligonucleotide to form a conjugate having a phosphoramidate linkage. In another variation of OLA described in Tobe et al. ((1996) Nucleic Acids Res 24: 3728), OLA combined with PCR permits typing of two alleles in a single microtiter well. By marking each of the allele-specific primers with a unique hapten, i.e. digoxigenin and fluorescein, each OLA reaction can be detected by using hapten specific antibodies that are labeled with different enzyme reporters, alkaline phosphatase or horseradish peroxidase. This system permits the detection of the two alleles using a high throughput format that leads to the production of two different colors.

Several methods have been developed to facilitate analysis of single nucleotide polymorphisms. In one embodiment, the single base polymorphism can be detected by using a specialized exonuclease-resistant nucleotide, as disclosed, e.g., in Mundy, C. R. (U.S. Pat. No.4,656,127). According to the method, a primer complementary to the allelic sequence immediately 3' to the polymorphic site is permitted to hybridize to a target molecule obtained from a particular animal or human. If the polymorphic site on the target molecule contains a nucleotide that is complementary to the particular exonuclease-resistant nucleotide derivative present, then that derivative will be incorporated onto the end of the hybridized primer. Such incorporation renders the primer resistant to exonuclease, and thereby permits its detection. Since the identity of the exonuclease-resistant derivative of the sample is known, a finding that the primer has become resistant to exonucleases reveals that the nucleotide present in the polymorphic site of the target molecule was complementary to that of the nucleotide derivative used in the reaction. This method has the advantage that it does not require the determination of large amounts of extraneous sequence data.

In another embodiment of the invention, a solution-based method is used for determining the identity of the nucleotide of a polymorphic site. Cohen, D. et al. (French Patent 2,650,840; PCT Appln. No. WO91/02087). As in the Mundy method of U.S. Pat. No. 4,656,127, a primer is employed that is complementary to allelic sequences immediately 3' to a polymorphic site. The method determines the identity of the nucleotide of that site using labeled dideoxynucleotide derivatives, which, if complementary to the nucleotide of the polymorphic site will become incorporated onto the terminus of the primer.

An alternative method, known as Genetic Bit Analysis or GBA™ is described by Goelet, P. et al. (PCT Appln. No. 92/15712). The method of Goelet, P. et al. uses mixtures of labeled terminators and a primer that is complementary to the sequence 3' to a polymorphic site. The labeled terminator that is incorporated is thus determined by, and complementary to, the nucleotide present in the polymorphic site of the target molecule being evaluated. In contrast to the method of Cohen et al. (French Patent 2,650,840; PCT Appln. No. WO91/02087) the method of Goelet, P. et al. is preferably a heterogeneous phase assay, in which the primer or the target molecule is immobilized to a solid phase.

Recently, several primer-guided nucleotide incorporation procedures for assaying polymorphic sites in DNA have been described (Komher, J. S. et al., Nucl. Acids. Res. 17:7779-7784 (1989); Sokolov, B. P., Nucl. Acids Res. 18:3671 (1990); Syvanen, A. -C., et al., Genomics 8:684-692 (1990); Kuppuswamy, M. N. et al., Proc. Natl. Acad. Sci. (U.S.A.) 88:1143-1147 (1991); Prezant, T. R. et al., Hum. Mutat. 1:159-164 (1992); Ugozzoli, L. et al., GATA 9:107-112 (1992); Nyren, P. et al., Anal. Biochem. 208:171-175 (1993)). These methods differ from GBA ™ in that they all rely on the incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvanen, A. -C., et al., Amer.J. Hum. Genet. 52:46-59 (1993)).

For mutations that produce premature termination of protein translation, the protein truncation test (PTT) offers an efficient diagnostic approach (Roest, et. al., (1993) Hum. Mol. Genet. 2:1719-21; van der Luijt, et. al., (1994) Genomics 20:1-4). For PTT, RNA is initially isolated from available tissue and reverse-transcribed, and the segment of interest is amplified by PCR. The products of reverse transcription PCR are then used as a template for nested PCR amplification with a primer that contains an RNA polymerase promoter and a sequence for initiating eukaryotic translation. After amplification of the region of interest, the unique motifs incorporated into the primer permit sequential *in vitro* transcription and translation of the PCR products. Upon sodium dodecyl sulfate-polyacrylamide gel electrophoresis of translation products, the appearance of truncated polypeptides signals the presence of a mutation that causes premature termination of translation. In a variation of this technique, DNA (as opposed to RNA) is used as a PCR template when the target region of interest is derived from a single exon.

Any cell type or tissue may be utilized to obtain nucleic acid samples for use in the diagnostics described herein. In a preferred embodiment, the DNA sample is obtained from a bodily fluid, e.g, blood, obtained by known techniques (e.g. venipuncture) or saliva. Alternatively, nucleic acid tests can be performed on dry samples (e.g. hair or skin). When using RNA or protein, the cells or tissues that may be utilized must express an IL-1 gene.

Diagnostic procedures may also be performed *in situ* directly upon tissue sections (fixed and/or frozen) of patient tissue obtained from biopsies or resections, such that no nucleic acid purification is necessary. Nucleic acid reagents may be used as probes and/or primers for such *in situ* procedures (see, for example, Nuovo, G.J., 1992, PCR in situ hybridization: protocols and applications, Raven Press, NY).

In addition to methods which focus primarily on the detection of one nucleic acid sequence, profiles may also be assessed in such detection schemes. Fingerprint profiles may be generated, for example, by utilizing a differential display procedure, Northern analysis and/or RT-PCR.

Also described are kits for detecting a predisposition for developing asthma.

This kit may contain one or more oligonucleotides, including 5' and 3' oligonucleotides that hybridize 5' and 3' to at least one allele of an IL-1 proinflammatory haplotype. PCR amplification oligonucleotides should hybridize between 25 and 2500 base pairs apart, preferably between about 100 and about 500 bases apart, in order to produce a PCR product of convenient size for subsequent analysis.

For use in a kit, oligonucleotides may be any of a variety of natural and/or synthetic compositions such as synthetic oligonucleotides, restriction fragments, cDNAs, synthetic peptide nucleic acids (PNAs), and the like. The assay kit and method may also employ labeled oligonucleotides to allow ease of identification in the assays. Examples of labels which may be employed include radio-labels, enzymes, fluorescent compounds, streptavidin, avidin, biotin, magnetic moieties, metal binding moieties, antigen or antibody moieties, and the like.

The kit may, optionally, also include DNA sampling means. DNA sampling means are well known to one of skill in the art and can include, but not be limited to substrates, such as filter papers, the AmpliCard™ (University of Sheffield, Sheffield, England S10 2JF; Tarlow, JW, et al., J. of Invest. Dermatol. 103:387-389 (1994)) and the like; DNA purification reagents such as Nucleon™ kits, lysis buffers, proteinase solutions and the like; PCR reagents, such as 10X reaction buffers, thermostable polymerase, dNTPs, and the like; and allele detection means such as the *Hinf*I restriction enzyme, allele specific oligonucleotides, degenerate oligonucleotide primers for nested PCR from dried blood.

### 4.2.2. Pharmacogenomics

Knowledge of the particular IL-1 polymorphisms that are predictive of sepsis, alone or in conjunction with information on other genetic defects contributing to a chronic obstructive airway disease (the genetic profile of the chronic obstructive airway disease) allows a customization of the therapy for a particular disease to the individual's genetic profile, the goal of "pharmacogenomics". For example, subjects having the IL-1B allele 2 (-511) and/or IL-1B allele 2 (+3954) are predisposed to developing a chronic obstructive airway disease or for progressing more rapidly or severely into a chronic obstructive airway disease. Thus, comparison of an individual's IL-1 proinflammatory profile to the population profile for a chronic obstructive airway disease, permits the selection or design of drugs that are expected to be safe and efficacious for a particular patient or patient population (i.e., a group of patients having the same genetic alteration).

The ability to target populations expected to show the highest clinical benefit, based on the IL-1B or disease genetic profile, can enable: 1) the repositioning of marketed asthma drugs with disappointing market results; 2) the rescue of asthma drug candidates whose clinical development has been discontinued as a result of safety or efficacy limitations, which are patient subgroup-specific; and 3) an accelerated and less costly development for asthma drug candidates and more optimal drug labeling (e.g. since the use of markers described herein are useful for optimizing effective dose).

Cells of a subject may also be obtained before and after administration of a therapeutic to detect the level of expression of genes other than IL-1, to verify that the therapeutic does not increase or decrease the expression of genes which could be deleterious. This can be done, e.g., by using the method of transcriptional profiling. Thus, mRNA from cells exposed in vivo to a therapeutic and mRNA from the same type of cells that were not exposed to the therapeutic could be reverse transcribed and hybridized to a chip containing DNA from numerous genes, to thereby compare the expression of genes in cells treated and not treated with the therapeutic.

The present invention is further illustrated by the following examples which should not be construed as limiting in any way.

The practice of the present invention will employ, unless otherwise indicated, conventional techniques, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. G.ait ed., 1984); Mullis et al. U.S. Patent No: 4,683,195; Nucleic Acid Hybridization(B. D. Hames & S. J. Higgins eds. 1984); Transcription And Translation (B. D. Hames & S. J. Higgins eds. 1984).

### EXAMPLE 1: Detection of IL-1B (+3954)

The screening of the single base variation (C/T) polymorphism at IL-1B base +3954 was conducted by PCR amplification of genomic templates. One mismatch was inserted in a primer to complete a *Taq*I site as a positive control. The polymorphic *Taq*I site is native. The following primers were produced in an ABI DNA synthesizer based on the genomic sequences (Clark et al., 1986; GENBANK X04500):
5' CTC AGG TGT CCT CGA AGA AAT CAA A 3' (SEQ ID No:1)
5' GCT TTT TTG CTG TGA GTC CCG 3' (SEQ ID No:2)

The PCR reaction conditions were as follows:
[95 C (2 minutes)] 1 cycle;
[95 C(1 minute), 67.5 C (1 minute), 74 C (1 minute)] 38 cycles; and
[72 C (8 minutes)] 1 cycle.

Restriction enzyme digestion was conducted at 60°C, for 8 hours. Sizing was by 8% PAGE. The digestion of the PCR product with *Taq I* yields a segment of 12 bp (the absence of which indicates incomplete digestion) and either two further segments of 85 and 97 bp (allele 1), or a single one of 182 bp (allele 2).

### EXAMPLE 2: Detection of IL-1B (-511)

The single base polymorphism (C/T) at position - 511 in the IL-1B gene was screened by PCR amplification of genomic templates, followed by RFLP (Restriction Fragment Length Polymorphism) analysis. The gene variation completes an *Ava* I restriction site in the most frequent allele, and a *Bsu 36 I* site in the rarer allele. Hence digestion of the PCR product with these enzymes provides efficient analysis of the IL-1B (-511) locus.

The following primers were produced in an ABI synthesizer based on the genomic sequence (Clark et al, 1986; Genbank X04500):
5' TGG CAT TGA TCT GGT TCA TC-3' (SEQ ID No:3)
5' GTT TAG GAA TCT TCC CAC TT-3' (SEQ ID No:4)
PCR conditions were as follows:
[95 C (1 minute)] I cycle
[95 C (1 minute)] 53 C (1 minute), 72 (1 minute)] 35 cycles
[72 C (5 minute)] 1 cycle.

Each PCR reaction was divided in two 25 µl aliquots; one was added to 3 units of *Ava* I, the other to 3.7 units of *Bsu* 36 I, in addition to 3 µl of the specific 10X restriction buffer. Digestion was at 37 °C overnight, sizing was by 9% PAGE. *Ava* I digestion produced 190 + 114 bp segments with allele 1, while allele 2 was uncut (304 bp). The *Bsu* 36 I digestion produced 190 + 114 bp fragments with allele 2, while allele 1 was uncut (304 bp). The restriction pattern obtained was inverted in the two aliquots (identifying homozygotes) or identical (identifying heterozygotes). This protocol provided efficient analysis of the IL-1B (-511) locus.

### Example 3 Association of IL-1B allele 2 (+3954) and IL-1B allele 2 (-511) With The Presence of Asthma in a Subject

The following study was conducted to evaluate whether there was an association between asthma and alleles found in the relevant regions of the IL-1B gene. One hundred six (106) asthma patients were recruited for the study. 251 North British white Caucasian non-asthmatic subjects were recruited as controls. All asthma patients fulfilled the ATS criteria for the definition of asthma *(*Amer Rev Respir Dis 1985, 132:180-182.), and where relevant had a PC20 methacholine of less than 4mg/mi. Asthma patients were clinically categorized as having either mild or severe asthma. Severe asthma was defined as those patients requiring more than 800mg/day of inhaled steroids. Asthma patients on beta-2 agonist alone were categorized as having mild asthma. Of the total number of asthma patients, 50 were mild asthmatics on beta 2 agonist alone (FEV1 92.5± 1.5% pred) and had a mean age of 26.5±0.9, and 56 were severe asthmatics on a regimen of at least 800 mg per day of inhaled steroids (FEV1 58.4±3.4% pred) with a mean age of 47.2±2.3. After informed consent was obtained, 10mls of venous blood was drawn and collected in EDTA-containing tubes from each patient. Total genomic DNA was extracted and allele frequencies were assessed in DNA extracted from the 106 patients. For IL-1B (+3954) 105 patients could be genotyped. 104 patients were genotyped for IL-1B (-511), For each DNA, a single PCR product spanning the relevant regions of the IL-1B gene was produced and analyzed as described in Example 1. The data were analyzed using the Chi square test to compare carriage of the rare allele (genotypes carrying at least one copy of allele 2 between cohorts). The results for IL-1B (+3954) are presented in the following Table 1 and the results for IL-1B (-511) are presented in the following Table 2.

**Table 1**

| **IL-1B (+3954)** | | | | |
|---|---|---|---|---|
| | Disease Severity | 1.1 | 1.2 | 2.2 |
| | MILD (N=50) | 28 | 17 | 5 |
| | SEVERE (N=55) | 26 | 24 | 5 |
| | CONTROLS (N=251) | 165 | 81 | 5 |
| Mild vs Severe | | Chi²=0.497 | p=0.48 | (N.S.) |
| "all" vs Control | | Chi²=6.402 | p=0.01 | O.R.=1.81 (95% |
| | | | | C.I.=1.14-2.88) |
| Severe vs Control | | Chi²=6.557 | p=0.01 | O.R.=2.14(95% |
| | | | | C.I.=1.19-3.86) |

**Table 2**

| **IL-1B (-511)** | | | | |
|---|---|---|---|---|
| Disease Severity | | 1.1 | 1.2 | 2.2 |
| | MILD (N=50) | 2 | 19 | 3 |
| | SEVERE (N=54) | 19 | 31 | 4 |
| | CONTROLS (N=251) | 89 | 129 | 33 |
| Severe vs Mild | | Chi²=4.541 | p=0.033 | O.R.=2.34 (95% |
| | | | | C.I.=1.06-5.16) |
| "all" vs Control | | Chi²=2.948 | p=0.086 | (NS) |

As evidenced by Tables 1 and 2, the presence of IL-1B allele 2 (+3954) and IL-1B allele 2 (-511) are significantly associated with clinical asthma. Further, the presence of at least one copy of allele 2 at the IL-1B (-511) locus was found to be associated with more severe disease.

## Claims

1. A method for determining a subject's increased risk of susceptibility to developing asthma in the subject, said method comprising the steps of
a) providing a nucleic acid sample obtained from blood or saliva or a sample from skin or hair from the subject; and
b) detecting at least IL-1B allele 2 (-511) containing a T at IL-1B base -511 or IL-1B allele 2 (+3954) containing a T at IL-1B base +3954 in said sample, wherein detection of said allele indicates that the patient has an increased risk of susceptibility to developing asthma.

2. A method of claim 1, wherein the allele is IL-1B allele 2 (-511) containing a T at IL-1B base -511.

3. A method of claim 2, wherein the detecting step comprises amplification using at least one primer selected from the group consisting of:

5' CTC AGG TGT CCT CGA AGA AAT CAA A3' (SEQ ID No: 1);

5' GCT TTT TTG CTG TGA GTC CCG 3' (SEQ ID No: 2)[?] .

4. A method of claim 1, wherein the allele is IL-1B allele 2 (+3954) containing a T at IL-1B base +3954.

5. A method of claim 4, wherein the detecting step comprises amplification using at least one primer selected from the group consisting of:

5'TGG CAT TGA TCT GGT TCA TC-3' (SEQ ID No:3);

5' GTT TAG GAA TCT TCC CAC TT-3' (SEQIDNo:4)

6. A method of claim 1, wherein the presence of said IL-1B allele 2 (-511) is indicative of an increased propensity for severe asthma.

7. A method of claim 1, wherein the detecting step further comprises amplification using at least one primer selected from the group consisting of

5'-CTC. AGC. AAC. ACT. CCT. AT-3' (SEQ ID NO. 5)
5'-TCC.TGG.TCT.GCA.GCT.AA-3'(SEQ ID NO. 6)
5'-CTA TCT GAG GAA CAA ACT AGT AGC-3' (SEQ ID NO. 7)
5'-TAG GAC ATT GCA CCT AGG GTT TGT-3' (SEQ ID NO. 8)
5'-AAG CTT GTT CTA CCA CCT GAA CTA GGC-3' (SEQ ID NO. 9)
5'-TTA CAT ATG AGC CTT CCA TG. -3' (SEQ ID NO. 10)

## Patentansprüche

1. Methode zum Bestimmen eines erhöhten Risikos einer Person einer Anfälligkeit für die Entwicklung von Asthma bei der Person, welche Methode die folgenden Schritte umfasst:
a) Bereitstellen einer Nukleinsäure-Probe, die aus Blut oder Speichel erhalten ist, oder einer Probe von Haut oder Haar von der Person; und
b) Nachweisen zumindest des IL-1 B-Allels 2 (-511), enthaltend ein T an der IL-1 B-Base -511 oder IL-1 B-Allel 2 (+3954), enthaltend ein T an der IL-1 B-Base +3954 in der Probe, wobei der Nachweis des Allels anzeigt, dass der Patient ein erhöhtes Risiko einer Anfälligkeit für die Entwicklung von Asthma aufweist.

2. Methode nach Anspruch 1, wobei das Allel das IL-1B-Allel 2 (-511), enthaltend ein T an der IL-1B-Base -511, ist.

3. Methode nach Anspruch 2, wobei der Nachweisschritt die Amplifikation unter Verwendung mindestens eines Primers umfasst, ausgewählt aus der Gruppe, bestehend aus:

5' CTC AGG TGT CCT CGA AGA AAT CAA A3' (SEQ ID Nr. 1);
5' GCT TTT TTG CTG TGA GTC CCG 3' (SEQ ID Nr. 2).

4. Methode nach Anspruch 1, wobei das Allel das IL-1B-Allel 2 (+3954), enthaltend ein T an der IL-1 B-Base +3954, ist.

5. Methode nach Anspruch 4, wobei der Nachweisschritt die Amplifikation unter Verwendung mindestens eines Primers umfasst, ausgewählt aus der Gruppe, bestehend aus:

5' TGG CAT TGA TCT GGT TCA TC-3' (SEQ ID Nr. 3);
5' GTT TAG GAA TCT TCC CAC TT-3' (SEQ ID Nr. 4)

6. Methode nach Anspruch 1, wobei das Vorhandensein des IL-1B-Allels 2 (-511) für eine erhöhte Neigung zu schwerem Asthma indikativ ist.

7. Methode nach Anspruch 1, wobei der Nachweisschritt außerdem die Amplifikation unter Verwendung mindestens eines Primers umfasst, ausgewählt aus der Gruppe, bestehend aus:

5'-CTC.AGC.AAC.ACT.CCT.AT-3' (SEQ ID Nr. 5)
5'-TCC.TGG.TCT.GCA.GCT.AA-3' (SEQ ID Nr. 6)
5'-CTA TCT GAG GAA CAA ACT AGT AGC-3' (SEQ ID Nr. 7)
5'-TAG GAC ATT GCA CCT AGG GTT TGT-3' (SEQ ID Nr. 8)
5'-AAG CTT GTT CTA CCA CCT GAA CTA GGC-3' (SEQ ID Nr. 9)
5'-TTA CAT ATG AGC CTT CCA TG.-3' (SEQ ID Nr. 10)

## Revendications

1. Procédé pour la détermination d'un risque accru chez un sujet d'une sensibilité au développement de l'asthme chez le sujet, ledit procédé comprenant les étapes consistant à :
a) se procurer un échantillon d'acide nucléique obtenu à partir du sang ou de la salive ou d'un échantillon provenant de la peau ou des cheveux du sujet ; et
b) détecter dans ledit échantillon au moins l'allèle 2 de Il-1B (-511) contenant une T au niveau de la base -511 de l'IL-1B ou l'allèle 2 de IL-1B (+3954) contenant une T au niveau de la base +3954 de l'IL-1B, la détection dudit allèle indiquant que le patient a un risque accru d'une sensibilité au développement de l'asthme.

2. Procédé selon la revendication 1, dans lequel l'allèle est l'allèle 2 de IL-1B (-511) contenant une T au niveau de la base -511 de l'IL-1B.

3. Procédé selon la revendication 2, dans lequel l'étape de détection comprend l'amplification au moyen d'au moins une amorce sélectionnée dans le groupe constitué de :

5′ CTC AGG TGT CCT CGA AGA AAT CAA A3′ (SEQ ID NO: 1) ;
5′ GCT TTT TTG CTG TGA GTC CCG 3′ (SEQ ID NO: 2).

4. Procédé selon la revendication 1, dans lequel l'allèle est l'allèle 2 de IL-1B (+3954) contenant une T au niveau de la base +3954 de l'IL-1B.

5. Procédé selon la revendication 4, dans lequel l'étape de détection comprend une amplification au moyen d'au moins une amorce sélectionnée dans le groupe constitué de :

5'TGG CAT TGA TCT GGT TCA TC-3' (SEQ ID NO: 3) ;
5' GTT TAG GAA TCT TCC CAC TT-3' (SEQ ID NO: 4)

6. Procédé selon la revendication 1, dans lequel la présence dudit allèle 2 de IL-1B (-511) indique une propension accrue à de l'asthme grave.

7. Procédé selon la revendication 1, dans lequel l'étape de détection comprend en outre une amplification au moyen d'au moins une amorce sélectionnée dans le groupe constitué de
